# FASCICULE DE BREVET EUROPEEN

(11) **EP 3 484 554 B1**
(45) Date de publication et mention de la délivrance du brevet: **09.12.2020**
(21) Numéro de dépôt: 17745798.3
(22) Date de dépôt: 27.06.2017
(51) Int. Cl.: A61M 5/315, A61M 5/00

(54) **DISPOSITIF MÉDICAL COMPORTANT UN EMBALLAGE ET UNE SERINGUE PRÉ-REMPLIE DISPOSÉE DANS L'EMBALLAGE**
MEDIZINISCHE VORRICHTUNG MIT EINEM GEHÄUSE UND EINER VORGEFÜLLTEN, IN DEM GEHÄUSE ANGEORDNETEN SPRITZE
MEDICAL DEVICE COMPRISING A PACKAGE AND A PRE-FILLED SYRINGE ARRANGED IN THE PACKAGE

(30) Priorité: 13.07.2016 FR 1656712
(43) Date de publication de la demande: 22.05.2019
(73) Titulaire: Laboratoire Aguettant, 69007 Lyon (FR)
(72) Inventeur: BONNEFOND, Guillaume, 69230 Saint Genis Laval (FR); GUYOT, Vincent, 69960 Corbas (FR); LAURENT, Philippe, 69600 Oullins (FR)
(74) Mandataire: Verriest, Philippe
(86) Numéro de dépôt international: PCT/FR2017/051705
(87) Numéro de publication internationale: WO 2018/011485

(56) Documents cités:
- WO-A1-2012/076510
- US-A- 2 421 495
- US-A- 4 973 318
- US-A- 5 417 326
- US-A1- 2008 110 917

## Description

La présente invention concerne un dispositif médical comportant une seringue pré-remplie et un emballage dans lequel est disposée la seringue pré-remplie.

Le document FR2896168 divulgue une seringue pré-remplie comportant :
- un corps de seringue délimitant en partie une chambre interne contenant un fluide à administrer à un patient,
- un piston monté coulissant dans le corps de seringue selon une direction de déplacement axiale,
- une tige de piston solidaire en translation avec le piston,
- un embout de raccordement comportant une partie de raccordement tubulaire destinée au passage du fluide à administrer,
- un obturateur obturant l'extrémité libre de la partie de raccordement tubulaire, l'obturateur étant relié à l'extrémité libre de la partie de raccordement tubulaire par une zone sécable, et
- un capuchon de protection monté sur l'embout de raccordement et comportant une partie d'accouplement couplée à l'obturateur de sorte qu'une rotation de la partie d'accouplement autour d'un axe de rotation parallèle à la direction d'extension de la partie de raccordement tubulaire entraîne une rupture de la zone sécable En outre, le document US 5,417,326 divulgue un dispositif médical comportant un emballage et une serinque pré-remplie selon le préambule de la revendication 1.

Une telle seringue pré-remplie est généralement disposée dans un emballage, également nommé blister, afin de préserver la stérilité de la seringue pré-remplie.

Lorsqu'une telle seringue pré-remplie est stockée pendant une longue durée avant son utilisation, souvent le piston adhère à la surface intérieure du corps de seringue. Il est alors nécessaire, pour un praticien souhaitant administrer le fluide contenu dans la seringue pré-remplie, d'actionner la tige de piston préalablement à la rupture de l'obturateur afin de déplacer le piston à l'intérieur du corps de seringue, et donc de le « décoller » du corps de seringue. Une telle étape constitue une étape d'activation de la seringue pré-remplie.

Cependant, lorsqu'un praticien oublie d'effectuer l'étape d'activation préalablement à la rupture de l'obturateur et au retrait du capuchon de protection, et la réalise donc après le raccordement de la seringue pré-remplie au cathéter relié au patient, l'application d'une pression élevée sur la tige de piston pour décoller le piston induit un déplacement soudain et non contrôlé du piston, et est donc susceptible de provoquer d'une part une administration douloureuse du fluide à administrer, et d'autre part l'administration d'une quantité de fluide différente de celle requise, ce qui pourrait être très préjudiciable, voire mortel, pour le patient selon la nature du fluide à administrer.

En outre, lorsqu'un praticien effectue l'étape d'activation après la rupture de l'obturateur et le retrait du capuchon de protection, mais avant le raccordement de la seringue pré-remplie au cathéter relié au patient, l'application d'une pression élevée sur la tige de piston pour décoller le piston induit une éjection soudaine d'une quantité du fluide à administrer hors de la seringue, et donc une perte de produit et un risque pour le patricien ou le patient d'être contaminé par le fluide éjecté.

La présente invention vise à remédier à ces inconvénients.

Le problème technique à la base de l'invention consiste donc à fournir un dispositif médical comportant une seringue pré-remplie, qui assure une administration fiable, sure et non dangereuse du fluide à administrer contenu dans la seringue pré-remplie, et qui participe à limiter les erreurs et les risques d'utilisation par les professionnels de santé.

A cet effet, la présente invention concerne un dispositif médical, comprenant :
- une seringue pré-remplie comportant un corps de seringue et un piston monté coulissant dans le corps de seringue selon une direction de déplacement axiale, le corps de seringue et le piston délimitant une chambre interne contenant un fluide à administrer à un patient ; et
- un emballage dans lequel est disposée la seringue pré-remplie, l'emballage comportant une première partie d'emballage et une deuxième partie d'emballage montées mobiles l'une par rapport à l'autre entre une configuration de stockage dans laquelle les première et deuxième parties d'emballage empêchent un retrait de la seringue pré-remplie hors de l'emballage, et une configuration d'ouverture dans laquelle les première et deuxième parties d'emballage autorisent un retrait de la seringue pré-remplie hors de l'emballage, le dispositif médical étant configuré de telle sorte qu'un déplacement des première et deuxième parties d'emballage de la configuration de stockage à la configuration d'ouverture entraîne un déplacement du piston de la seringue pré-remplie d'une première position de piston à une deuxième position de piston.

Une telle configuration du dispositif médical selon la présente invention garantit une activation de la seringue pré-remplie avant son retrait hors de l'emballage, et assure donc une administration fiable et non dangereuse du fluide à administrer contenu dans la seringue pré-remplie.

En particulier, une telle configuration du dispositif médical selon la présente invention simplifie l'étape de préparation de la seringue et assure donc un gain de temps en situation d'urgence. Ainsi, le dispositif médical selon la présente invention limite grandement les risques de blessure du personnel hospitalier et les risques d'erreur de dosage, notamment en situation d'urgence.

Le dispositif médical peut en outre présenter une ou plusieurs des caractéristiques suivantes, prises seules ou en combinaison.

Selon un mode de réalisation de l'invention, l'emballage est allongé et s'étend selon une direction d'extension.

Selon un mode de réalisation de l'invention, la première partie d'emballage comporte un logement de réception dans lequel est disposée au moins en partie la seringue pré-remplie, et une ouverture de passage débouchant dans le logement de réception et destinée au passage de la seringue pré-remplie, la deuxième partie d'emballage étant configurée pour obturer au moins en partie l'ouverture de passage et pour empêcher un retrait de la seringue pré-remplie hors du logement de réception lorsque les première et deuxième parties d'emballage sont dans la configuration de stockage, et étant configurée pour libérer au moins en partie l'ouverture de passage et pour autoriser un retrait de la seringue pré-remplie hors du logement de réception lorsque les première et deuxième parties d'emballage sont dans la configuration d'ouverture.

Selon un mode de réalisation de l'invention, les première et deuxième parties d'emballage sont configurées pour occuper une configuration intermédiaire d'activation, l'emballage étant configuré de telle sorte qu'un déplacement des première et deuxième parties d'emballage de la configuration de stockage à la configuration intermédiaire d'activation entraîne un déplacement du piston dans le corps de seringue selon une première direction axiale.

Selon un mode de réalisation de l'invention, l'emballage est configuré de telle sorte qu'un déplacement des première et deuxième parties d'emballage de la configuration intermédiaire d'activation à la configuration d'ouverture entraîne un déplacement du piston dans le corps de seringue selon une deuxième direction axiale opposée à la première direction axiale.

Selon un mode de réalisation de l'invention, l'emballage est configuré de telle sorte qu'un déplacement des première et deuxième parties d'emballage de la configuration intermédiaire d'activation à la configuration d'ouverture entraîne un déplacement du piston de la deuxième position de piston à la première position de piston.

Selon un mode de réalisation de l'invention, l'une des première et deuxième parties d'emballage comporte une première surface de poussée axiale configurée pour transmettre un effort de poussée au piston, et l'autre des première et deuxième parties d'emballage comporte une deuxième surface de poussée axiale configurée pour transmettre un effort de poussée au corps de seringue, l'emballage étant configuré de telle sorte qu'un déplacement des première et deuxième parties d'emballage de la configuration de stockage à la configuration intermédiaire d'activation entraîne un rapprochement axial des première et deuxième surfaces de poussée axiales de manière à provoquer un déplacement relatif du corps de seringue et du piston.

Selon un mode de réalisation de l'invention, l'emballage est configuré de telle sorte qu'un déplacement des première et deuxième parties d'emballage de la configuration intermédiaire d'activation à la configuration d'ouverture entraîne un éloignement axial des première et deuxième surfaces de poussée axiales.

Selon un mode de réalisation de l'invention, la seringue pré-remplie comporte une tige de piston solidaire en translation avec le piston et montée mobile par rapport au corps de seringue selon la direction de déplacement axiale, et l'une des première et deuxième parties d'emballage est configurée pour coopérer avec la tige de piston et déplacer la tige de piston selon la direction de déplacement axiale lors du déplacement des première et deuxième parties d'emballage de la configuration de stockage à la configuration d'ouverture.

Selon un mode de réalisation de l'invention, la première surface de poussée axiale est configurée pour transmettre un effort de poussée à la tige de piston.

Selon un mode de réalisation de l'invention, la première partie d'emballage comporte au moins un organe de retenue configuré pour retenir la seringue pré-remplie sur la première partie d'emballage. Ces dispositions permettent notamment de pourvoir présenter de manière aseptique la seringue pré-remplie à un utilisateur, et d'aider l'utilisateur à la saisir en maintenant une asepsie.

Selon un mode de réalisation de l'invention, l'au moins un organe de retenue est configuré pour coopérer avec la tige de piston ou le corps de seringue.

Selon un mode de réalisation de l'invention, l'au moins un organe de retenue est configuré pour déplacer la tige de piston à distance du corps de seringue lorsque les première et deuxième partie d'emballage sont déplacées de la configuration intermédiaire d'activation à la configuration d'ouverture, de manière à déplacer le piston de la première position de piston à la deuxième position de piston.

Selon un mode de réalisation de l'invention, la seringue pré-remplie comporte un embout de raccordement comportant une partie de raccordement tubulaire destinée au passage du fluide à administrer, et un obturateur configuré pour obturer l'extrémité libre de la partie de raccordement tubulaire, l'obturateur étant relié à l'extrémité libre de la partie de raccordement tubulaire par une zone sécable.

Selon un mode de réalisation de l'invention, la seringue pré-remplie comporte un capuchon de protection monté sur l'embout de raccordement.

Selon un mode de réalisation de l'invention, le capuchon de protection comprend une partie d'accouplement couplée à l'obturateur de sorte qu'une rotation de la partie d'accouplement autour d'un axe de rotation parallèle à la direction d'extension de la partie de raccordement tubulaire entraîne une rupture de la zone sécable.

Selon un mode de réalisation de l'invention, la deuxième partie d'emballage comporte au moins un élément de retenue configuré pour retenir le capuchon de protection, l'emballage étant configuré de telle sorte qu'un déplacement des première et deuxième parties d'emballage de la configuration de stockage à la configuration d'ouverture entraîne une retenue du capuchon de protection sur la deuxième partie d'emballage par l'au moins un élément de retenue et une rupture de la zone sécable reliant l'obturateur et la partie de raccordement tubulaire. En particulier, la retenue du capuchon de protection par l'au moins un élément de retenue lors du déplacement des première et deuxième parties d'emballage de la configuration de stockage à la configuration d'ouverture induit la rupture de la zone sécable reliant l'obturateur et la partie de raccordement tubulaire.

Selon un mode de réalisation de l'invention, le corps de seringue est solidaire en rotation avec la première partie d'emballage.

Selon un mode de réalisation de l'invention, le capuchon de protection comporte au moins un élément d'entraînement configuré pour coopérer avec l'au moins un élément de retenue, la deuxième partie d'emballage et le capuchon de protection étant montés mobile en rotation l'un par rapport à l'autre de telle sorte que l'au moins un élément de retenue est apte à occuper une première position angulaire dans laquelle l'au moins un élément de retenue est décalé angulairement de l'au moins un élément d'entraînement, et une deuxième position angulaire dans laquelle l'au moins un élément de retenue coopère avec l'au moins un élément d'entraînement de manière à rompre la zone sécable et à retenir le capuchon de protection, l'emballage étant configuré de telle sorte qu'un déplacement des première et deuxième parties d'emballage de la configuration de stockage à la configuration d'ouverture entraîne un déplacement de l'au moins un élément de retenue de la première position angulaire à la deuxième position angulaire.

Selon un mode de réalisation de l'invention, l'élément d'entraînement est une ailette ou nervure d'entraînement, et peut par exemple s'étendre sensiblement longitudinalement.

Selon un mode de réalisation de l'invention, l'au moins un élément de retenue comporte une surface de butée radiale configurée pour limiter la course angulaire de l'au moins un élément de retenue par rapport au capuchon de protection, et une surface de butée axiale configurée pour retenir le capuchon de protection sur la deuxième partie d'emballage.

Selon un mode de réalisation de l'invention, la deuxième partie d'emballage comporte deux éléments de retenue diamétralement opposés, et le capuchon de protection comporte deux éléments d'entraînement diamétralement opposés configurés chacun pour coopérer avec l'un respectif des deux éléments de retenue. Avantageusement, les deux éléments de retenue forment un système de retenue à baïonnette.

Selon un mode de réalisation de l'invention, l'emballage comporte des moyens de guidage configurés pour guider l'une des première et deuxième parties d'emballage par rapport à l'autre des première et deuxième parties d'emballage selon un premier mouvement hélicoïdal dans un premier sens d'enroulement lors d'un déplacement des première et deuxième parties d'emballage de la configuration de stockage à la configuration intermédiaire d'activation, et selon un deuxième mouvement hélicoïdal dans un deuxième sens d'enroulement inversé par rapport au premier sens d'enroulement lors d'un déplacement des première et deuxième parties d'emballage de la configuration intermédiaire d'activation vers la configuration d'ouverture.

Selon un mode de réalisation de l'invention, les moyens de guidage comportent un ergot de guidage prévu sur l'une des première et deuxième parties d'emballage, et une rainure de guidage prévue sur l'autre des première et deuxième parties d'emballage, l'ergot de guidage étant monté coulissant dans la rainure de guidage.

Selon un mode de réalisation de l'invention, la rainure de guidage comporte une première partie de rainure enroulée en hélice selon un premier sens d'enroulement, et une deuxième partie de rainure enroulée en hélice selon un deuxième sens d'enroulement inversé par rapport au premier sens d'enroulement. Avantageusement, la deuxième partie de rainure s'étend dans le prolongement de la première partie de rainure.

Selon un mode de réalisation de l'invention, la rainure de guidage comporte une troisième partie de rainure configurée pour autoriser un retrait de l'ergot de guidage hors de la rainure de guidage. Ces dispositions permettent un montage amovible de l'une des première et deuxième parties d'emballage sur l'autre des première et deuxième parties d'emballage.

Selon un mode de réalisation de l'invention, la troisième partie de rainure est rectiligne et s'entend sensiblement parallèlement à la direction d'extension de la seringue pré-remplie, et plus particulièrement de l'emballage.

Selon un mode de réalisation de l'invention, les première et deuxième parties d'emballage sont montées pivotantes l'une par rapport à l'autre autour d'un axe de pivotement entre la configuration de stockage et la configuration d'ouverture.

Selon un mode de réalisation de l'invention, la deuxième partie d'emballage comporte un organe d'actionnement comportant une surface de came configurée pour déplacer la tige de piston d'une première position de tige à une deuxième position de tige lors du pivotement des première et deuxième parties d'emballage de la configuration de stockage à la configuration d'ouverture.

Selon un mode de réalisation de l'invention, la première partie d'emballage comporte des moyens d'immobilisation configurés pour immobiliser axialement le corps de seringue sur la première partie d'emballage.

Selon un mode de réalisation de l'invention, les moyens d'immobilisation comportent au moins une première surface de butée axiale et une deuxième surface de butée axiale disposées de part et d'autre d'une collerette d'appui prévue sur le corps de seringue, et par exemple sur une portion d'extrémité proximale du corps de seringue.

Selon un mode de réalisation de l'invention, au moins l'une des première et deuxième parties d'emballage comporte des moyens de préhension, tels que des crantages, configurés pour faciliter le déplacement des première et deuxième parties d'emballage de la configuration de stockage à la configuration d'ouverture.

Selon un mode de réalisation de l'invention, l'emballage comporte des moyens de verrouillage mobiles entre une position de verrouillage dans laquelle les moyens de verrouillage empêchent un déplacement des première et deuxième parties d'emballage de la configuration de stockage à la configuration d'ouverture, et une position de déverrouillage dans laquelle les moyens de verrouillage autorisent un déplacement des première et deuxième parties d'emballage de la configuration de stockage à la configuration d'ouverture.

Selon un mode de réalisation de l'invention, les moyens de verrouillage comportent un logement de verrouillage prévu sur l'une des première et deuxième parties d'emballage, et au moins une languette de verrouillage prévue sur l'autre des première et deuxième parties d'emballage et pourvue d'un élément de verrouillage, la languette de verrouillage étant élastiquement déformable entre une position de verrouillage dans laquelle l'élément de verrouillage est configuré pour coopérer avec le logement de verrouillage et une position de déverrouillage dans laquelle l'élément de verrouillage est configuré pour libérer le logement de verrouillage.

Selon un mode de réalisation de l'invention, la première partie d'emballage comporte au moins un organe de sollicitation configuré pour solliciter la tige de piston vers la première position de tige. Avantageusement, l'au moins un organe de sollicitation est élastiquement déformable. L'au moins un organe de sollicitation peut par exemple venir de matière avec la première partie d'emballage.

Selon un mode de réalisation de l'invention, l'emballage comporte au moins un élément de blocage configuré pour empêcher un déplacement des première et deuxième parties d'emballage de la configuration d'ouverture à la configuration de stockage. Une telle configuration permet d'indiquer à un utilisateur si l'emballage a déjà été ouvert ou non, et donc de garantir à un utilisateur la stérilité de la seringue pré-remplie disposée dans l'emballage.

L'au moins un élément de blocage est par exemple une patte de blocage élastiquement déformable entre une position de libération dans laquelle la patte de blocage autorise un déplacement des première et deuxième parties d'emballage de la configuration de stockage à la configuration d'ouverture, et une position de blocage dans laquelle la patte de blocage empêche un déplacement des première et deuxième parties d'emballage de la configuration d'ouverture à la configuration de stockage.

Selon un mode de réalisation de l'invention, la deuxième partie d'emballage est montée de manière amovible sur la première partie d'emballage.

Selon un mode de réalisation de l'invention, le dispositif médical comporte un dessiccant et/ou un absorbeur d'oxygène disposé dans l'emballage.

Selon un mode de réalisation de l'invention, l'emballage comporte une ouverture d'accrochage destinée au passage d'un organe de support d'un présentoir pour dispositifs médicaux.

Selon un mode de réalisation de l'invention, la seringue pré-remplie est disposée de manière stérile dans l'emballage. De ce fait, la seringue pré-remplie peut être manipulée dans un environnement de soins aseptique.

Selon un mode de réalisation de l'invention, la seringue pré-remplie est réalisée en matière synthétique. Avantageusement, la seringue pré-remplie est obtenue par moulage, et l'obturateur vient de moulage en une seule pièce avec l'embout de raccordement et le corps de seringue.

Selon un mode de réalisation de l'invention, l'embout de raccordement est du type Luer ou Luer-Lock
Selon un mode de réalisation de l'invention, le capuchon de protection est fixé sur l'obturateur, notamment par soudage, collage ou interférence mécanique.

Selon un mode de réalisation de l'invention, le dispositif médical comporte au moins un élément de communication, tel qu'une puce RFID ou une puce NFC, configuré pour coopérer avec un dispositif de communication, tel qu'un ordiphone, un terminal, etc.

Selon un mode de réalisation de l'invention, l'emballage peut être pourvu d'un code d'identification permettant d'identifier la seringue pré-remplie disposée dans l'emballage. Le code d'identification peut par exemple être disposé sur une étiquette, et prendre la forme d'un code-barres ou d'un code datamatrix, ou indiquer le nom du fluide à administrer contenu dans la seringue pré-remplie. Ces dispositions permettent d'éviter les erreurs d'administration par le personnel de santé.

Selon un mode de réalisation de l'invention, l'emballage présente une section transversale non circulaire, et par exemple rectangulaire, carrée ou ellipsoïdale. Ces dispositions évident le roulement de l'emballage sur un support horizontal.

Selon un mode de réalisation de l'invention, l'emballage comporte au moins deux surfaces d'appui latérales sensiblement planes et opposées l'une à autre. Ces dispositions permettent notamment un empilement de différents dispositifs médicaux selon la présente invention, ce qui réduit l'espace de stockage de ces dispositifs dans les zones de rangements de médicaments, telles que dans les armoires à médicaments des offices des personnels de soin.

Selon un mode de réalisation de l'invention, l'emballage est rigide. Ces dispositions assurent à l'emballage une résistance à la performation et aux chocs élevée, et ainsi assure une protection grandement améliorée de la seringue pré-remplie. De plus, une telle configuration de l'emballage permet de disposer la seringue pré-remplie après utilisation au moins en partie dans l'emballage, et donc de faciliter sa mise au rebut.

Selon un mode de réalisation de l'invention, l'emballage est opaque, c'est-à-dire ne laisse pas passer la lumière. Ces dispositions assurent une protection de la seringue pré-remplie contre le rayonnement lumineux pendant le stockage du dispositif médical.

Selon un mode de réalisation de l'invention, le dispositif médical comporte une tige de piston montée de manière amovible sur l'emballage, la tige de piston étant configurée pour être reliée au piston après retrait de la seringue pré-remplie hors de l'emballage et pour déplacer le piston selon la direction de déplacement axiale.

Selon un mode de réalisation de l'invention, le dispositif médical comporte au moins une aiguille d'injection montée de manière amovible sur l'emballage.

Selon un mode de réalisation de l'invention, en configuration de stockage, les première et deuxième parties d'emballage sont assemblées l'une à l'autre de manière à former une barrière, et en particulier de manière à former une barrière aux microorganismes et aux gaz.

Selon un mode de réalisation de l'invention, au moins l'une, et par exemple chacune, des première et deuxième parties d'emballage est pourvue d'au moins un orifice de passage destiné au passage d'un fluide de stérilisation, tel que de la vapeur d'eau ou de l'oxyde d'éthylène. Avantageusement, l'au moins un orifice de passage est recouvert par un film perméable au fluide de stérilisation et imperméable aux bactéries, tel qu'un film en Tyvek (marque déposée). De cette façon, le dispositif médical selon la présente invention peut être stérilisé en phase terminale, c'est-à-dire à la fin du procédé d'assemblage.

Selon un mode de réalisation de l'invention, l'emballage comporte des premiers moyens d'assemblage et des deuxièmes moyens d'assemblage complémentaires des premiers moyens d'assemblage, les premiers moyens d'assemblage du dispositif médical étant configurés pour coopérer avec les deuxièmes moyens d'assemblage d'un dispositif médical adjacent de manière à permettre l'assemblage des deux dispositifs médicaux. Ces dispositions facilitent le stockage des dispositifs médicaux et réduit en outre l'espace de stockage de ces dispositifs dans les zones de rangements de médicaments.

Selon un mode de réalisation de l'invention, les premiers moyens d'assemblage sont disposés à l'opposé des deuxièmes moyens d'assemblage.

Selon un mode de réalisation de l'invention, la première partie d'emballage est reliée au piston. Par exemple, la première partie d'emballage peut comporter une partie de liaison reliée au piston, directement ou via la tige de piston, et s'étendant au moins en partie dans le corps de seringue.

Selon un mode de réalisation de l'invention, la partie de liaison et le piston sont formés d'un seul tenant. Néanmoins, la partie de liaison pourrait être fixée sur le piston notamment par vissage, collage ou soudure.

De toute façon l'invention sera bien comprise à l'aide de la description qui suit en référence aux dessins schématiques annexés représentant, à titre d'exemples non limitatifs, plusieurs formes d'exécution de ce dispositif médical.
Figure 1 est une vue en perspective d'un dispositif médical selon un premier mode de réalisation de l'invention.
Figure 2 est une vue en perspective coupée selon un plan longitudinal du dispositif médical de la figure 1.
Figure 3 est une vue éclatée en perspective du dispositif médical de la figure 1.
Figures 4 à 6 sont des vues en perspective coupées selon des plans transversaux du dispositif médical de la figure 1, montrant des première et deuxième parties d'emballage du dispositif médical dans différentes positions angulaires relatives.
Figure 7 est une vue en perspective du dispositif médical de la figure 1 dans une configuration d'ouverture.
Figure 8 est une vue en perspective coupée selon un plan longitudinal du dispositif médical de la figure 1 dans une configuration intermédiaire d'activation.
Figure 9 est une vue en perspective d'un dispositif médical selon un deuxième mode de réalisation de l'invention.
Figure 10 est une vue en perspective du dispositif médical de la figure 9, montrant les première et deuxième parties d'emballage du dispositif médical dans une configuration d'ouverture.
Figure 11 est une vue en perspective coupée selon un plan longitudinal du dispositif médical de la figure 9.
Figure 12 est une vue en perspective d'un dispositif médical selon un troisième mode de réalisation de l'invention.
Figure 13 est une vue en perspective du dispositif médical de la figure 12, montrant les première et deuxième partie d'emballage d'un tel dispositif médical dans une configuration d'ouverture.
Figure 14 est une vue partielle de dessus du dispositif médical de la figure 12.
Figure 15 est une vue partielle en perspective, à l'échelle agrandie, du dispositif médical de la figure 12.
Figures 16 à 18 sont des vues partielles en coupe du dispositif médical de la figure 12, montrant l'emballage d'un tel dispositif médical dans différentes positions.
Figures 19 et 20 sont des vues partielles en perspective coupées selon des plans longitudinaux du dispositif médical de la figure 12, montrant l'emballage d'un tel dispositif médical dans différentes positions.
Figure 21 est une vue à l'échelle agrandie d'un détail de la figure 18.
Figure 22 est une vue en perspective de deux dispositifs médicaux selon un quatrième mode de réalisation de l'invention.

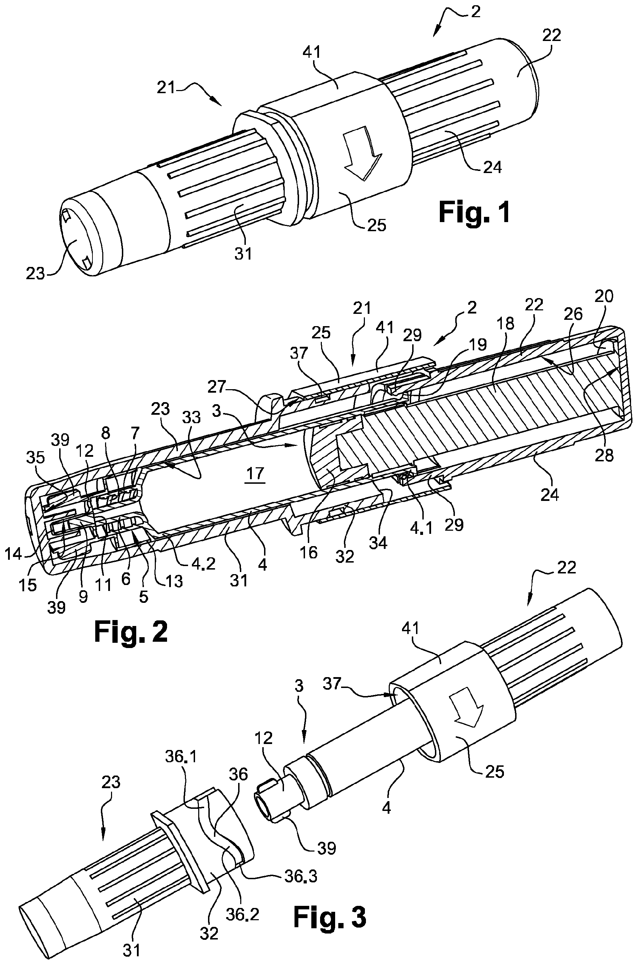

Les figures 1 à 8 représentent un dispositif médical 2 selon un premier mode de réalisation de l'invention.

Comme montré plus particulièrement sur les figures 2 et 3, le dispositif médical 2 comporte une seringue pré-remplie 3 comportant un corps de seringue 4 tubulaire et de forme globalement cylindrique. Le corps de seringue 4 comporte une extrémité proximale 4.1 et une extrémité distale 4.2.

La seringue pré-remplie 3 comporte également un embout de raccordement 5 de type Luer-lock disposé au niveau de l'extrémité distale 4.2 du corps de seringue 4. L'embout de raccordement 5 comprend une partie de raccordement tubulaire 6 reliée fluidiquement au volume intérieur du corps de seringue 4, et un manchon de verrouillage 7 disposé coaxialement à la partie de raccordement tubulaire 6 et entourant cette dernière. Le manchon de verrouillage 7 comporte de préférence un filetage interne 8.

La seringue pré-remplie 3 comporte de plus un obturateur 9 relié par une zone sécable 11 à l'extrémité libre de la partie de raccordement 6. La zone sécable 11 entre la partie de raccordement 6 et l'obturateur 9 est réalisée avantageusement par un amincissement annulaire de la matière le long de la ligne de raccordement entre l'extrémité libre de la partie de raccordement 6 et l'obturateur 9. Une telle configuration de la zone sécable 11 permet une séparation aisée de l'obturateur 9 et de la partie de raccordement 6.

Le corps de seringue 4, l'embout de raccordement 5 et l'obturateur 9 sont par exemple réalisés en matière synthétique et en une seule pièce par moulage.

La seringue pré-remplie 3 comporte en outre un capuchon de protection 12 montée de manière amovible sur l'embout de raccordement 5 afin de préserver la stérilité de l'embout de raccordement 5. Le capuchon de protection 12 comporte avantageusement une partie de montage 13 configurée pour coopérer avec la surface extérieure du manchon de verrouillage 7. La partie de montage 13 s'étend de préférence jusqu'à proximité du corps de seringue 4, et présente avantageusement un diamètre externe correspondant sensiblement au diamètre externe du corps de seringue 4.

Le capuchon de protection 12 comprend également une partie d'accouplement 14 solidaire de la partie de montage 13. Selon le mode de réalisation représenté sur les figures 1 à 8, la partie d'accouplement 14 comporte un logement d'accouplement 15 débouchant dans un volume interne délimité par la partie de montage 13. Le logement d'accouplement 15 est agencé pour coopérer avec l'obturateur 9 de sorte que la rotation de la partie d'accouplement 14 autour d'un axe de rotation confondu avec l'axe d'extension de la partie de raccordement 6 entraîne en rotation l'obturateur 9 et provoque la rupture de la zone sécable 11.

Selon le mode de réalisation représenté sur les figures 1 à 8, l'obturateur 9 et le logement d'accouplement 15 présentent chacun une forme tronconique. Ces dispositions permettent un emmanchement à force de l'obturateur 9 dans le logement d'accouplement 15 de sorte que, après rupture de la zone sécable 11, l'obturateur 9 soit maintenu dans le logement d'accouplement 15 et ne puisse pas tomber au sol. Selon un autre mode de réalisation de l'invention, l'obturateur 9 et le logement d'accouplement 15 pourrait présenter d'autres formes, tels que des formes polygonales complémentaires, par exemple hexagonales.

La seringue pré-remplie 3 comporte également un piston 16 monté coulissant à l'intérieur du corps de seringue 4 suivant l'axe longitudinal de celui-ci. Le corps de seringue 4 et le piston 16 délimitent ainsi une chambre interne 17 contenant un fluide à administrer à un patient, par exemple une solution médicamenteuse.

La seringue pré-remplie 3 comporte de plus une tige de piston 18, formant poussoir, montée mobile par rapport au corps de seringue 4 selon une direction de déplacement axiale. La tige de piston 18 est reliée au piston 16, et est solidaire en translation avec le piston 16. La surface externe du corps de seringue 4 comporte avantageusement une collerette d'appui 19 située au niveau de l'extrémité proximale 4.1 du corps de seringue 4, et sur laquelle viennent s'appuyer les doigts de l'utilisateur lorsqu'une poussée est exercée par ce dernier sur une partie d'appui 20 prévue à l'extrémité de la tige de piston 18 opposée au piston 16.

Le dispositif médical 2 comporte en outre un emballage 21 dans lequel est disposée, de préférence de manière stérile, la seringue pré-remplie 3. Avantageusement, l'emballage 21 est allongé et s'étend selon une direction d'extension.

L'emballage 21 comporte plus particulièrement une première partie d'emballage 22 et une deuxième partie d'emballage 23. Les première et deuxième parties d'emballage 22, 23 sont avantageusement rigides et opaques, et peuvent être réalisées par exemple en matière plastique.

La première partie d'emballage 22 comporte une portion de préhension 24 de forme globalement tubulaire, et une portion de montage 25 également de forme globalement tubulaire et s'étendant coaxialement à la portion de préhension 24. La portion de préhension 24 et la portion de montage 25 délimitent un logement de réception 26 dans lequel sont disposés la tige de piston 18 et une partie du corps de seringue 4.

La portion de montage 25 comporte une ouverture de passage 27 débouchant dans le logement de réception 26, et destinée au passage de la tige de piston 18 et du corps de seringue 4 afin de permettre leur insertion et leur retrait dans et hors du logement de réception 26.

La portion de préhension 24 comporte une surface de poussée axiale 28 délimitant en partie le logement de réception 26, et sur laquelle prend appui la partie d'appui 20 de la tige de piston 18. La surface de poussée axiale 28 est plus particulièrement configurée pour transmettre un effort de poussée à la tige de piston 18.

Selon le premier mode de réalisation de l'invention, la première partie d'emballage 22 comporte également une pluralité d'organes de retenue 29 configurés pour coopérer avec la collerette d'appui 19 du corps de seringue 4 de manière à retenir le corps de seringue 4 sur la première partie d'emballage 22. Chaque organe de retenue 29 se présente avantageusement sous la forme d'un doigt de retenue élastiquement déformable, et s'étend de préférence parallèlement à la direction d'extension du corps de seringue 4. Chaque organe de retenue 29 s'étend par exemple à distance et le long de la surface intérieure de la portion de montage 25.

Il convient d'être noté que la première partie d'emballage 22 est configurée de telle sorte que le corps de seringue 4 est solidaire en rotation avec la première partie d'emballage 22.

La deuxième partie d'emballage 23 comporte également une portion de préhension 31 de forme globalement tubulaire, et une portion de montage 32 également de forme globalement tubulaire. La portion de préhension 31 et la portion de montage 32 délimitent un logement interne 33 dans lequel sont disposés le capuchon de protection 12 et une partie du corps de seringue 4.

La portion de montage 32 comporte une ouverture de passage 34 débouchant dans le logement interne 33, et destinée au passage du capuchon de protection 12 et du corps de seringue 4 afin de permettre leur insertion et leur retrait dans et hors du logement interne 33. La portion de montage 32 est configurée pour coopérer avec la portion de montage 25 de la première partie d'emballage 22, et plus particulièrement pour coopérer avec la surface intérieure de la portion de montage 25.

La portion de préhension 31 comporte plus particulièrement une surface de poussée axiale 35 sur laquelle prend appui le capuchon de protection 12. La surface de poussée axiale 35 est plus particulièrement configurée pour transmettre un effort de poussée au corps de seringue 4 via le capuchon de protection 12.

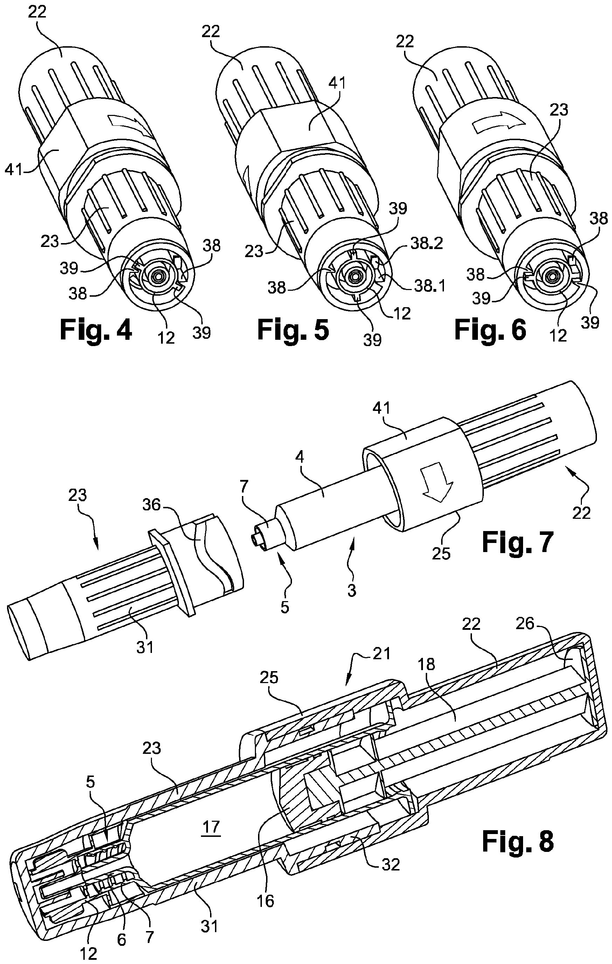

Comme montré notamment sur les figures 4 à 6, les première et deuxième parties d'emballage 22, 23 sont montées mobiles l'une par rapport à l'autre, et sont aptes à occuper notamment :
- une configuration de stockage (voir les figures 1 et 4) dans laquelle les première et deuxième parties d'emballage 22, 23 empêchent un retrait de la seringue pré-remplie 3 hors de l'emballage 21,
- une configuration intermédiaire d'activation (voir les figures 5 et 8) dans laquelle le piston 16 est activé,
- une configuration intermédiaire de rupture (voir la figure 6) dans laquelle la zone ruptible 11 est rompue, et
- une configuration d'ouverture (voir la figure 7) dans laquelle les première et deuxième parties d'emballage 22, 23 autorisent un retrait de la seringue pré-remplie 3 hors de l'emballage 21.

L'emballage 21 comporte des moyens de guidage configurés pour guider la première partie d'emballage 22 par rapport à la deuxième partie d'emballage 23 :
- selon un premier mouvement hélicoïdal dans un premier sens d'enroulement lors du déplacement des première et deuxième parties d'emballage 22, 23 de la configuration de stockage à la configuration intermédiaire d'activation,
- selon un deuxième mouvement hélicoïdal dans un deuxième sens d'enroulement inversé par rapport au premier sens d'enroulement lors du déplacement des première et deuxième parties d'emballage 22, 23 de la configuration intermédiaire d'activation à la configuration intermédiaire de rupture, et
- selon un mouvement axial lors du déplacement des première et deuxième parties d'emballage 22, 23 de la configuration intermédiaire de rupture à la configuration d'ouverture.

Les moyens de guidage comportent plus particulièrement une rainure de guidage 36 prévue sur la surface extérieure de la portion de montage 31 de la deuxième partie d'emballage 23, et un ergot de guidage 37 prévu sur la surface intérieure de la portion de montage 25 de la première partie d'emballage 22 et monté coulissant dans la rainure de guidage 36. La rainure de guidage 36 comporte une première partie de rainure 36.1 enroulée en hélice selon un premier sens d'enroulement, et une deuxième partie de rainure 36.2 s'étendant dans le prolongement de la première partie de rainure 63.1 et étant enroulée en hélice selon un deuxième sens d'enroulement inversé par rapport au premier sens d'enroulement.

Avantageusement, la rainure de guidage 36 comporte une troisième partie de rainure 36.3 s'entendant parallèlement à la direction d'extension de la deuxième partie d'emballage 23 et débouchant au niveau de l'extrémité libre de la portion de montage 31. La troisième partie de rainure 36.3 est plus particulièrement configurée pour autoriser un retrait de l'ergot de guidage 37 hors de la rainure de guidage 36, de manière à permettre une séparation des première et deuxième parties d'emballage 22, 23.

L'emballage 21 est configuré de telle sorte qu'un déplacement des première et deuxième parties d'emballage 22, 23 de la configuration de stockage à la configuration intermédiaire d'activation entraîne un rapprochement des surfaces de poussée axiales 28, 35, et donc un rapprochement de la partie d'appui 20 et de la collerette d'appuil9. Ainsi, l'emballage 21 est plus particulièrement configuré de telle sorte qu'un déplacement des première et deuxième parties d'emballage 22, 23 de la configuration de stockage à la configuration intermédiaire d'activation entraîne un déplacement du piston 16 dans le corps de seringue 4 en direction du capuchon de protection 12, et d'une première position de piston, dite de repos, à une deuxième position de piston, dite activée. Une telle configuration du dispositif médical 2 selon le premier mode de réalisation de l'invention garantit donc une activation de la seringue pré-remplie 3 avant son retrait hors de l'emballage 21, et assure ainsi une administration fiable et non douloureuse du fluide contenu dans la chambre interne 17 de la seringue pré-remplie 3.

Il convient d'être noté qu'un déplacement des première et deuxième parties d'emballage 22, 23 de la configuration intermédiaire d'activation à la configuration d'ouverture entraîne un éloignement axial des première et deuxième surfaces de poussée axiales 28, 35.

De façon avantageuse, comme cela est montré sur les figures 2 et 4 à 6, la deuxième partie d'emballage 23 comporte deux éléments de retenue 38 disposés dans le logement interne 33 et s'étendant à partir de la surface intérieure de la portion de préhension 31. Avantageusement, les deux éléments de retenue 38 sont diamétralement opposés, et forme un système de retenue à baïonnette. Ainsi, chaque élément de retenue 38 comporte une surface de butée radiale 38.1 et une surface de butée axiale 38.2.

Le capuchon de protection 12 comporte en outre deux éléments d'entraînement 39 diamétralement opposés, et configurés pour coopérer chacun avec l'un respectif des deux éléments de retenue 38. Avantageusement, chaque élément d'entraînement 38 se présente sous la forme d'une ailette ou nervure d'entraînement, et s'étend parallèlement à la direction d'extension de la seringue pré-remplie 3.

Comme montré plus particulièrement sur les figures 4 à 6, la deuxième partie d'emballage 23 et le capuchon de protection 12 sont montés mobile en rotation l'un par rapport à l'autre de telle sorte que chaque élément de retenue 38 est apte à occuper une première position angulaire dans laquelle ledit élément de retenue 38 est décalé angulairement de l'élément d'entraînement 39 respectif, et une deuxième position angulaire dans laquelle ledit élément de retenue 38 coopère avec l'élément d'entraînement 39 respectif, et plus précisément avec la surface de butée radiale respective 38.1 qui est configurée pour limiter la course angulaire dudit élément de retenue 38 par rapport au capuchon de protection 12 et la surface de butée axiale respective 38.2 qui est configurée pour retenir le capuchon de protection 12.

Ainsi, l'emballage 21 est également configuré de telle sorte qu'un déplacement des première et deuxième parties d'emballage 22, 23 de la configuration de stockage à la configuration d'ouverture entraîne un déplacement des éléments de retenue 38 de leur première position angulaire à leur deuxième position angulaire, et plus précisément une rupture de la zone sécable 11 reliant l'obturateur 9 et la partie de raccordement tubulaire 6 et une retenue du capuchon de protection 12 sur la deuxième partie d'emballage 23. Ces dispositions permettre d'assurer un retrait du capuchon de protection 12 de l'embout de raccordement 5 et une rupture de la zone sécable 11 avant le retrait de la seringue pré-remplie 3 hors de l'emballage 21. Il en résulte un geste facilité pour le praticien.

Il doit être noté que les éléments de retenue 38 sont positionnés de telle sorte que la rupture de la zone sécable 11 intervienne après l'activation du piston 16, et donc pendant le déplacement des première et deuxième parties d'emballage 22, 23 de la configuration intermédiaire d'activation à la configuration d'ouverture.

Selon le premier mode de réalisation représenté sur les figures 1 à 8, la première partie d'emballage 22 comporte avantageusement deux surfaces d'appui latérales 41 sensiblement planes et opposées l'une à autre. Ces dispositions permettent d'une part d'éviter un roulement de l'emballage 21 sur un support horizontal, et d'autre part d'assurer un empilement aisé de différents dispositifs médicaux 2 selon le premier mode de réalisation, ce qui réduit l'espace de stockage de ces dispositifs dans les zones de rangements de médicaments, telles que dans les armoires à médicaments des offices des personnels de soin.

Il convient également d'être noté que la première partie d'emballage 22 pourrait être pourvue d'un ou de plusieurs orifice(s) de passage débouchant dans le logement de réception 26 et destiné(s) au passage d'un fluide de stérilisation, tel que de la vapeur d'eau ou de l'oxyde d'éthylène. De même, la deuxième partie d'emballage 23 pourrait être pourvue d'un ou de plusieurs orifice(s) de passage débouchant dans le logement interne 33 et destiné(s) au passage d'un fluide de stérilisation. Avantageusement, chaque orifice de passage pourrait être recouvert par un film perméable au fluide de stérilisation et imperméable aux bactéries, tel qu'un film en Tyvek (marque déposée).

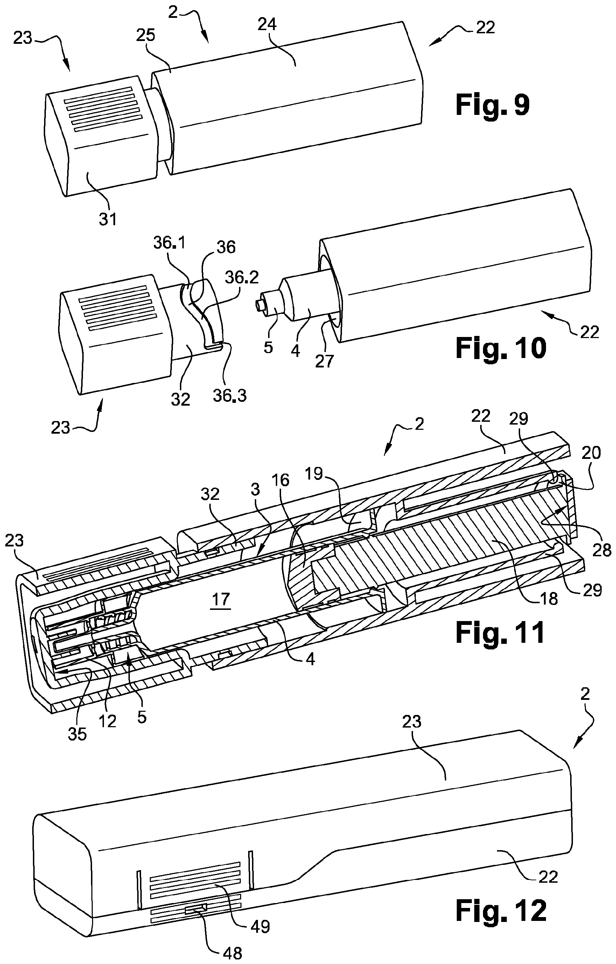

Les figures 9 à 11 représentent un dispositif médical 2 selon un deuxième mode de réalisation qui diffère du premier mode de réalisation essentiellement en ce que les organes de retenue 29 sont configurés pour coopérer avec la partie d'appui 20 de la tige de piston 18, et pour déplacer axialement la tige de piston 18 à distance du corps de seringue 4 lorsque les première et deuxième partie d'emballage 22, 23 sont déplacées de la configuration intermédiaire d'activation à la configuration d'ouverture.

L'emballage 21 est plus précisément configuré de telle sorte qu'un déplacement des première et deuxième parties d'emballage 22, 23 de la configuration intermédiaire d'activation à la configuration d'ouverture entraîne un déplacement du piston 16 de la deuxième position de piston à la première position de piston. Ces dispositions permettent de repositionner le piston 16 dans sa position de repos, et donc d'éviter une expulsion de fluide hors de la seringue pré-remplie 3 lors de la rupture de la zone sécable 11.

En outre, selon le deuxième mode de réalisation de l'invention, l'emballage 21 présente une section rectangulaire.

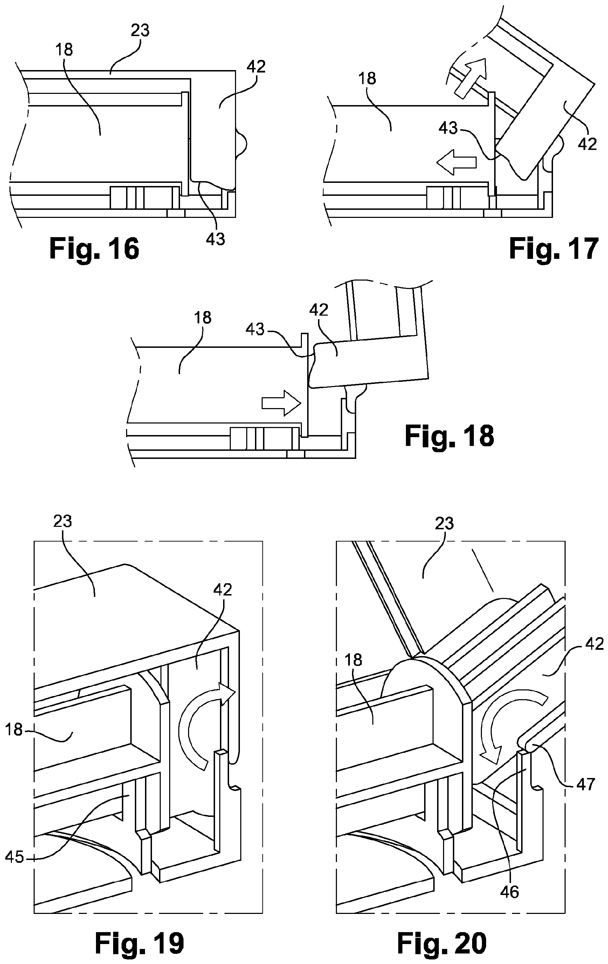

Les figures 12 à 21 représentent un dispositif médical 2 selon un troisième mode de réalisation qui diffère du premier mode de réalisation essentiellement en ce que les première et deuxième parties d'emballage 22, 23 sont montées pivotantes l'une par rapport à l'autre autour d'un axe de pivotement transversal à la direction d'extension de l'emballage 21, et en ce que la deuxième partie d'emballage 23 comporte un organe d'actionnement 42 comportant une surface de came 43 configurée pour déplacer la tige de piston 18 d'une première position de tige (voir la figure 16) à une deuxième position de tige (voir la figure 17) lors du pivotement des première et deuxième parties d'emballage 22, 23 de la configuration de stockage à la configuration intermédiaire d'activation.

Selon ce troisième mode de réalisation, la première partie d'emballage 22 comporte des moyens d'immobilisation configurés pour immobiliser axialement le corps de seringue 4 sur la première partie d'emballage 22. Avantageusement, les moyens d'immobilisation comportent deux rainures d'immobilisation 44 disposées de part et d'autre de la seringue pré-remplie 3 et dans lesquelles est reçue la collerette d'appui 19 du corps de seringue 4. Chaque rainure d'immobilisation 44 est délimitée par une première surface de butée axiale 44.1 et une deuxième surface de butée axiale 44.2 disposées de part et d'autre de la collerette d'appui 19. De tels moyens d'immobilisation permettent d'assurer un déplacement relatif de la tige de piston 18, et donc du piston 16, par rapport au corps de seringue 4 lors du déplacement des première et deuxième parties d'emballage 22, 23 de la configuration de stockage à la configuration intermédiaire d'activation.

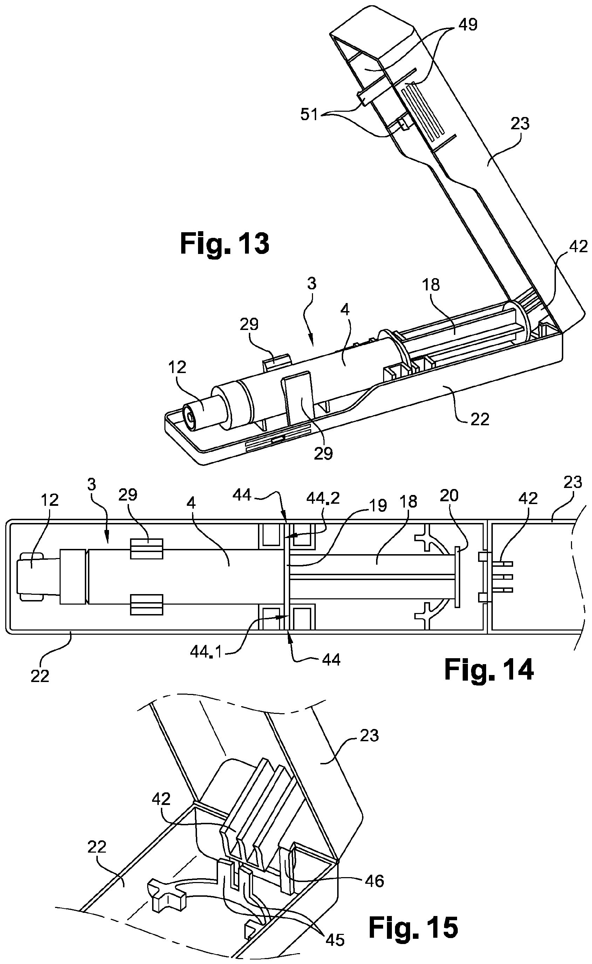

Selon ce troisième mode de réalisation, la première partie d'emballage 22 comporte également un ou plusieurs organes de sollicitation 45 (voir la figure 15) configuré(s) pour solliciter la tige de piston 18 vers la première position de tige lors du pivotement des première et deuxième parties d'emballage 22, 23 de la configuration intermédiaire d'activation à la configuration d'ouverture. Ces dispositions permettent d'assurer un repositionnement du piston 16 dans la première position de piston après le déplacement des première et deuxième parties d'emballage 22, 23 dans leur configuration d'ouverture.

Avantageusement, le ou chaque organe de sollicitation 45 est élastiquement déformable, et peut se présenter sous la forme d'une patte de sollicitation apte à coopérer avec la partie d'appui 20 de la tige de piston 18.

Selon ce troisième mode de réalisation, l'emballage 21 comporte en outre un élément de blocage 46 (voir notamment les figures 19 à 21) configuré pour empêcher un déplacement des première et deuxième parties d'emballage 22, 23 de la configuration d'ouverture à la configuration de stockage. Une telle configuration permet de garantir à un utilisateur la stérilité de la seringue pré-remplie 3 d'un dispositif médical 2 selon le troisième mode de réalisation de l'invention.

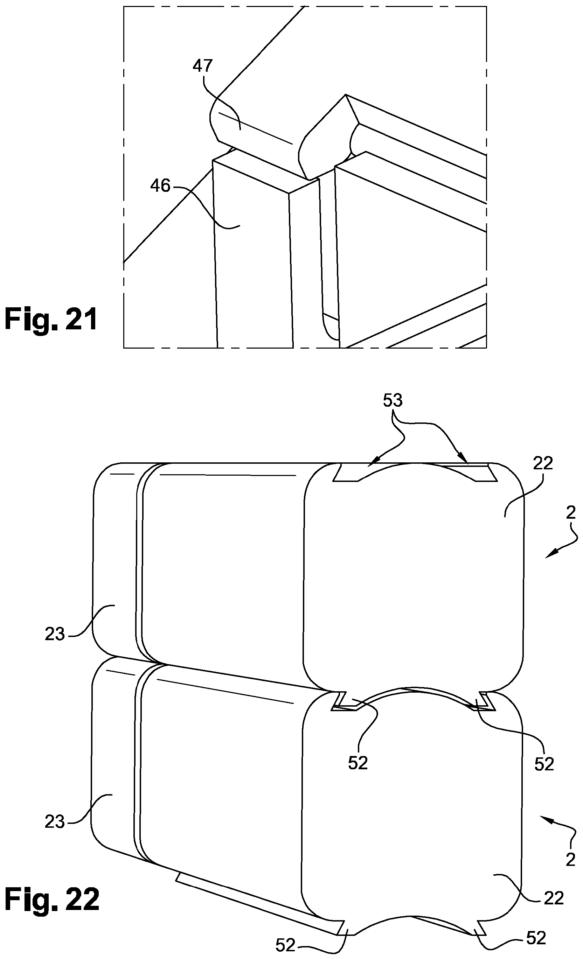

Par exemple, l'élément de blocage 46 peut être prévu sur la première partie d'emballage 22 et se présenter sous la forme d'une patte de blocage élastiquement déformable entre une position de libération dans laquelle la patte de blocage autorise un déplacement des première et deuxième parties d'emballage 22, 23 de la configuration de stockage à la configuration d'ouverture, et une position de blocage (voir les figures 20 et 21) dans laquelle la patte de blocage empêche un déplacement des première et deuxième parties d'emballage 22, 23 de la configuration d'ouverture à la configuration de stockage. Avantageusement, l'élément de blocage 46 est configuré pour coopérer avec un ergot de blocage 47 prévu sur la deuxième partie d'emballage 23.

De façon avantageuse, l'emballage 21 comporte de plus des moyens de verrouillage mobiles entre une position de verrouillage (voir la figure 12) dans laquelle les moyens de verrouillage empêchent un déplacement des première et deuxième parties d'emballage 22, 23 de la configuration de stockage à la configuration d'ouverture, et une position de déverrouillage dans laquelle les moyens de verrouillage autorisent un déplacement des première et deuxième parties 22, 23 de la configuration de stockage à la configuration d'ouverture.

Les moyens de verrouillage comportent par exemple au moins un logement de verrouillage 48 prévu sur la première partie d'emballage 22, et au moins une languette de verrouillage 49 prévue sur la deuxième partie d'emballage 23 et pourvue d'un élément de verrouillage 51. La ou chaque languette de verrouillage 49 est élastiquement déformable entre une position de verrouillage dans laquelle l'élément de verrouillage 51 respectif est configuré pour coopérer avec le logement de verrouillage 48 respectif, et une position de déverrouillage dans laquelle l'élément de verrouillage 51 respectif est configuré pour libérer le logement de verrouillage 48 respectif. De préférence, un utilisateur peut déformer la ou chaque languette de verrouillage 49 vers sa position de déverrouillage en exerçant une pression sur ladite languette de verrouillage 49.

Il convient d'être noté que, selon le troisième mode de réalisation de l'invention, l'emballage 21 n'est pas configuré de telle sorte qu'un déplacement des première et deuxième parties d'emballage 22, 23 entraîne une rupture de la zone sécable 11 et le retrait du capuchon de protection 12. Ainsi, selon ce mode de réalisation, le praticien devra déplacer en rotation le capuchon de protection 12 par rapport au corps de seringue 4, à l'aide des organes d'entraînement 39, pour rompre la zone ruptible 11 et retirer le capuchon de protection 12.

La figure 22 représente des dispositifs médicaux 2 selon un quatrième mode de réalisation de l'invention qui diffère du premier mode de réalisation essentiellement en ce que chaque emballage 21 comporte des premiers moyens d'assemblage et des deuxièmes moyens d'assemblage complémentaires des premiers moyens d'assemblage et disposés à l'opposé des premiers moyens d'assemblage, les premiers moyens d'assemblage d'un dispositif médical 2 étant configurés pour coopérer avec les deuxièmes moyens d'assemblage d'un dispositif médical 2 adjacent de manière à permettre l'assemblage des deux dispositifs médicaux adjacents.

Les premiers et deuxièmes moyens d'assemblage pourraient par exemple respectivement comporter une ou plusieurs nervures d'assemblage 52 et une ou plusieurs rainures d'assemblage 53. Néanmoins, les premiers et deuxièmes moyens d'assemblage pourraient prendre de toutes autres formes, et par exemple comporter des ergots ou des languettes d'assemblage configurés pour coopérer avec des logements d'assemblage complémentaires.

Il convient d'être noté que les premiers et deuxièmes moyens d'assemblage pourraient être prévus uniquement sur la première partie d'emballage 22, uniquement sur la deuxième partie d'emballage 23, ou encore sur les première et deuxième parties d'emballage.

Selon un mode de réalisation de l'invention non représenté sur les figures, le dispositif médical 2 pourrait comporter un dessiccant et/ou un absorbeur d'oxygène disposé dans l'emballage 21.

Selon un mode de réalisation de l'invention non représenté sur les figures, l'emballage 21 pourrait comporter une ouverture d'accrochage destinée au passage d'un organe de support d'un présentoir pour dispositifs médicaux, et permettant ainsi la disposition de l'emballage 21 sur un tel présentoir.

Selon un mode de réalisation de l'invention non représenté sur les figures, le dispositif médical 2 pourrait comporter un élément de communication, tel qu'une puce RFID ou une puce NFC, configuré pour coopérer avec un dispositif de communication, tel qu'un ordiphone, un terminal, etc. L'élément de communication serrait avantageusement disposé sur ou dans l'emballage 21.

Selon un mode de réalisation de l'invention, l'emballage 21 pourrait être pourvu d'un code d'identification permettant d'identifier la seringue pré-remplie 3 disposée dans l'emballage 21. Le code d'identification pourrait par exemple être disposé sur une étiquette, et prendre la forme d'un code-barres ou d'un code datamatrix.

Comme il va de soi, l'invention ne se limite pas aux seules formes d'exécution de ce dispositif médical, décrites ci-dessus à titre d'exemples, elle en embrasse au contraire toutes les variantes de réalisation.

## Revendications

1. Dispositif médical (2), comprenant :
- une seringue pré-remplie (3) comportant un corps de seringue (4) et un piston (16) monté coulissant dans le corps de seringue (4) selon une direction de déplacement axiale, le corps de seringue (4) et le piston (16) délimitant une chambre interne (17) contenant un fluide à administrer à un patient ; et
- un emballage (21) dans lequel est disposée la seringue pré-remplie (3), l'emballage (21) comportant une première partie d'emballage (22) et une deuxième partie d'emballage (23) montées mobiles l'une par rapport à l'autre entre une configuration de stockage dans laquelle les première et deuxième parties d'emballage (22, 23) empêchent un retrait de la seringue pré-remplie (4) hors de l'emballage (21), et une configuration d'ouverture dans laquelle les première et deuxième parties d'emballage (22, 23) autorisent un retrait de la seringue pré-remplie (3) hors de l'emballage (21),
**caractérisé en ce que** le dispositif médical (2) est configuré de telle sorte qu'un déplacement des première et deuxième parties d'emballage (22, 23) de la configuration de stockage à la configuration d'ouverture entraîne un déplacement du piston (16) de la seringue pré-remplie (3) d'une première position de piston à une deuxième position de piston.

2. Dispositif médical (2) selon la revendication 1, dans lequel la première partie d'emballage (22) comporte un logement de réception (26) dans lequel est disposée au moins en partie la seringue pré-remplie (3), et une ouverture de passage (27) débouchant dans le logement de réception (26) et destinée au passage de la seringue pré-remplie (3), la deuxième partie d'emballage (23) étant configurée pour obturer au moins en partie l'ouverture de passage (27) et pour empêcher un retrait de la seringue pré-remplie (3) hors du logement de réception (26) lorsque les première et deuxième parties d'emballage (22, 23) sont dans la configuration de stockage, et étant configurée pour libérer au moins en partie l'ouverture de passage (27) et pour autoriser un retrait de la seringue pré-remplie (3) hors du logement de réception (26) lorsque les première et deuxième parties d'emballage (22, 23) sont dans la configuration d'ouverture.

3. Dispositif médical (2) selon la revendication 1 ou 2, dans lequel les première et deuxième parties d'emballage (22, 23) sont configurées pour occuper une configuration intermédiaire d'activation, l'emballage (21) étant configuré de telle sorte qu'un déplacement des première et deuxième parties d'emballage (22, 23) de la configuration de stockage à la configuration intermédiaire d'activation entraîne un déplacement du piston (16) dans le corps de seringue (4) selon une première direction axiale.

4. Dispositif médical (2) selon la revendication 3, dans lequel l'emballage (21) est configuré de telle sorte qu'un déplacement des première et deuxième parties d'emballage (22, 23) de la configuration intermédiaire d'activation à la configuration d'ouverture entraîne un déplacement du piston (16) dans le corps de seringue (4) selon une deuxième direction axiale opposée à la première direction axiale.

5. Dispositif médical (2) selon la revendication 3 ou 4, dans lequel l'une des première et deuxième parties d'emballage (22, 23) comporte une première surface de poussée axiale (28) configurée pour transmettre un effort de poussée au piston (16), et l'autre des première et deuxième parties d'emballage (22, 23) comporte une deuxième surface de poussée axiale (35) configurée pour transmettre un effort de poussée au corps de seringue (4), l'emballage (21) étant configuré de telle sorte qu'un déplacement des première et deuxième parties d'emballage (22, 23) de la configuration de stockage à la configuration intermédiaire d'activation entraîne un rapprochement axial des première et deuxième surfaces de poussée axiales (28, 35) de manière à provoquer un déplacement relatif du corps de seringue (4) et du piston (16).

6. Dispositif médical (2) selon l'une quelconque des revendications 1 à 5, dans lequel la seringue pré-remplie (3) comporte une tige de piston (18) solidaire en translation avec le piston (16) et montée mobile par rapport au corps de seringue (4) selon la direction de déplacement axiale, et l'une des première et deuxième parties d'emballage (22, 23) est configurée pour déplacer la tige de piston (18) selon la direction de déplacement axiale lors du déplacement des première et deuxième parties d'emballage (22, 23) de la configuration de stockage à la configuration d'ouverture.

7. Dispositif médical (2) selon l'une quelconque des revendications 1 à 6, dans lequel la première partie d'emballage (22) comporte au moins un organe de retenue (29) configuré pour retenir la seringue pré-remplie (3) sur la première partie d'emballage (22).

8. Dispositif médical (2) selon l'une quelconque des revendications 1 à 7, dans lequel la seringue pré-remplie (3) comporte un embout de raccordement (5) comportant une partie de raccordement tubulaire (6) destinée au passage du fluide à administrer, et un obturateur (9) configuré pour obturer une extrémité libre de la partie de raccordement tubulaire (6), l'obturateur (9) étant relié à l'extrémité libre de la partie de raccordement tubulaire (6) par une zone sécable (11).

9. Dispositif médical (2) selon la revendication 8, dans lequel la seringue pré-remplie (3) comporte un capuchon de protection (12) monté sur l'embout de raccordement (5).

10. Dispositif médical (2) selon la revendication 9, dans lequel le capuchon de protection (12) comprend une partie d'accouplement (15) couplée à l'obturateur (9) de sorte qu'une rotation de la partie d'accouplement (15) autour d'un axe de rotation parallèle à la direction d'extension de la partie de raccordement tubulaire (6) entraîne une rupture de la zone sécable (11).

11. Dispositif médical (2) selon la revendication 10, dans lequel la deuxième partie d'emballage (23) comporte au moins un élément de retenue (38) configuré pour retenir le capuchon de protection (12), l'emballage (21) étant configuré de telle sorte qu'un déplacement des première et deuxième parties d'emballage (22, 23) de la configuration de stockage à la configuration d'ouverture entraîne une rupture de la zone sécable (11) reliant l'obturateur (9) et la partie de raccordement tubulaire (6) et une retenue du capuchon de protection (12) sur la deuxième partie d'emballage (23).

12. Dispositif médical (2) selon la revendication 11, dans lequel le capuchon de protection (12) comporte au moins un élément d'entraînement (39) configuré pour coopérer avec l'au moins un élément de retenue (38), la deuxième partie d'emballage (23) et le capuchon de protection (12) étant montés mobile en rotation l'un par rapport à l'autre de telle sorte que l'au moins un élément de retenue (38) est apte à occuper une première position angulaire dans laquelle l'au moins un élément de retenue (38) est décalé angulairement de l'au moins un élément d'entraînement (39), et une deuxième position angulaire dans laquelle l'au moins un élément de retenue (38) coopère avec l'au moins un élément d'entraînement (39) de manière à rompre la zone sécable (11) et à retenir le capuchon de protection (12), l'emballage (21) étant configuré de telle sorte qu'un déplacement des première et deuxième parties d'emballage (22, 23) de la configuration de stockage à la configuration d'ouverture entraîne un déplacement de l'au moins un élément de retenue (38) de la première position angulaire à la deuxième position angulaire.

13. Dispositif médical (2) selon la revendication 3 ou l'une quelconque des revendications 4 à 12 lorsqu'elle dépend de la revendication 3, dans lequel l'emballage (21) comporte des moyens de guidage configurés pour guider l'une des première et deuxième parties d'emballage (22, 23) par rapport à l'autre des première et deuxième parties d'emballage (22, 23) selon un premier mouvement hélicoïdal dans un premier sens d'enroulement lors d'un déplacement des première et deuxième parties d'emballage (22, 23) de la configuration de stockage à la configuration intermédiaire d'activation, et selon un deuxième mouvement hélicoïdal dans un deuxième sens d'enroulement inversé par rapport au premier sens d'enroulement lors d'un déplacement des première et deuxième parties d'emballage (22, 23) de la configuration intermédiaire d'activation vers la configuration d'ouverture.

14. Dispositif médical (2) selon la revendication 13, dans lequel les moyens de guidage comportent un ergot de guidage (37) prévu sur l'une des première et deuxième parties d'emballage (22, 23), et une rainure de guidage (36) prévue sur l'autre des première et deuxième parties d'emballage (22, 23), l'ergot de guidage (37) étant monté coulissant dans la rainure de guidage (36).

15. Dispositif médical (2) selon l'une quelconque des revendications 1 à 10, dans lequel les première et deuxième parties d'emballage (22, 23) sont montées pivotantes l'une par rapport à l'autre autour d'un axe de pivotement entre la configuration de stockage et la configuration d'ouverture.

16. Dispositif médical (2) selon les revendications 6 et 15, dans lequel la deuxième partie d'emballage (23) comporte un organe d'actionnement (42) comportant une surface de came (43) configurée pour déplacer la tige de piston (18) d'une première position de tige à une deuxième position de tige lors du pivotement des première et deuxième parties d'emballage (22, 23) de la configuration de stockage à la configuration d'ouverture.

17. Dispositif médical (2) selon la revendication 15 ou 16, dans lequel la première partie d'emballage (22) comporte des moyens d'immobilisation configurés pour immobiliser axialement le corps de seringue (4) sur la première partie d'emballage (22).

18. Dispositif médical (2) selon l'une quelconque des revendications 1 à 17, dans lequel la deuxième partie d'emballage (23) est montée de manière amovible sur la première partie d'emballage (22).

19. Dispositif médical (2) selon l'une quelconque des revendications 1 à 18, lequel comporte un dessiccant et/ou un absorbeur d'oxygène disposé dans l'emballage (21).

20. Dispositif médical (2) selon l'une quelconque des revendications 1 à 19, dans lequel la seringue pré-remplie (3) est disposée de manière stérile dans l'emballage (21).

21. Dispositif médical (2) selon l'une quelconque des revendications 1 à 20, dans lequel l'emballage (21) comporte des premiers moyens d'assemblage et des deuxièmes moyens d'assemblage complémentaires des premiers moyens d'assemblage, les premiers moyens d'assemblage du dispositif médical (2) étant configurés pour coopérer avec les deuxièmes moyens d'assemblage d'un dispositif médical adjacent de manière à permettre l'assemblage des deux dispositifs médicaux.

## Patentansprüche

1. Medizinische Vorrichtung (2), umfassend:
- eine vorgefüllte Spritze (3), umfassend einen Spritzenkörper (4) und einen Kolben (16), der gleitend im Spritzenkörper (4) gemäß einer axialen Verschieberichtung montiert ist, wobei der Spritzenkörper (4) und der Kolben (16) eine innere Kammer (17) begrenzen, die ein Fluid enthält, das einem Patienten verabreicht werden soll; und
- eine Verpackung (21), in der die vorgefüllte Spritze (3) angeordnet ist, wobei die Verpackung (21) einen ersten Verpackungsteil (22) und einen zweiten Verpackungsteil (23) umfasst, die beweglich mit Bezug zueinander zwischen einer Lagerkonfiguration, in der der erste und zweite Verpackungsteil (22, 23) ein Herausziehen der vorgefüllten Spritze (4) aus der Verpackung (21) verhindern, und einer Öffnungskonfiguration montiert ist, in der der erste und zweite Verpackungsteil (22, 23) ein Herausziehen der vorgefüllten Spritze (3) aus der Verpackung (21) ermöglichen,
**dadurch gekennzeichnet dass** die medizinische Vorrichtung (2) derart konfiguriert ist, dass die Verschiebung des ersten und zweiten Verpackungsteils (22, 23) aus der Lagerkonfiguration in die Öffnungskonfiguration eine Verschiebung des Kolbens (16) der vorgefüllten Spritze (3) aus einer ersten Kolbenposition in eine zweite Kolbenposition verursacht.

2. Medizinische Vorrichtung (2) nach Anspruch 1, wobei der erste Verpackungsteil (22) einen Aufnahmeraum (26) umfasst, in dem mindestens teilweise die vorgefüllte Spritze (3) angeordnet ist, und eine Durchgangsöffnung (27), die in den Aufnahmeraum (26) mündet und zum Durchgang der vorgefüllten Spritze (3) ausgelegt ist, wobei der zweite Verpackungsteil (23) konfiguriert ist, um mindestens teilweise die Durchgangsöffnung (27) zu verschließen, und um ein Herausziehen der vorgefüllten Spritze (3) aus dem Aufnahmeraum (26) zu verhindern, wenn sich der erste und zweite Verpackungsteil (22, 23) in der Lagerkonfiguration befinden, und konfiguriert ist, um mindestens teilweise die Durchgangsöffnung (27) freizusetzen, und um ein Herausziehen der vorgefüllten Spritze (3) aus dem Aufnahmeraum (26) zu ermöglichen, wenn sich der erste und zweite Verpackungsteil (22, 23) in der Öffnungskonfiguration befinden.

3. Medizinische Vorrichtung (2) nach Anspruch 1 oder 2, wobei der erste und zweite Verpackungsteil (22, 23) konfiguriert sind, um eine Zwischen-Aktivierungskonfiguration einzunehmen, wobei die Verpackung (21) derart konfiguriert ist, dass eine Verschiebung des ersten und zweiten Verpackungsteils (22, 23) aus der Lagerkonfiguration in die Zwischen-Aktivierungskonfiguration eine Verschiebung des Kolbens (16) im Spritzenkörper (4) gemäß einer ersten axialen Richtung verursacht.

4. Medizinische Vorrichtung (2) nach Anspruch 3, wobei die Verpackung (21) derart konfiguriert ist, dass eine Verschiebung des ersten und zweiten Verpackungsteils (22, 23) aus der Zwischen-Aktivierungskonfiguration in die Öffnungskonfiguration eine Verschiebung des Kolbens (16) im Spritzenkörper (4) gemäß einer zweiten axialen Richtung gegenüber der ersten axialen Richtung verursacht.

5. Medizinische Vorrichtung (2) nach Anspruch 3 oder 4, wobei einer des ersten und zweiten Verpackungsteils (22, 23) eine erste axiale Druckfläche (28) umfasst, die konfiguriert ist, um eine Druckkraft auf den Kolben (16) zu übertragen, und der andere des ersten und zweiten Verpackungsteils (22, 23) eine zweite axiale Druckfläche (35) umfasst, die konfiguriert ist, um eine Druckkraft auf den Spritzenkörper (4) zu übertragen, wobei die Verpackung (21) derart konfiguriert ist, dass eine Verschiebung des ersten und zweiten Verpackungsteils (22, 23) aus der Lagerkonfiguration in die Zwischen-Aktivierungskonfiguration eine axiale Annäherung der ersten und zweiten axialen Druckfläche (28, 35) verursacht, um eine relative Verschiebung des Spritzenkörpers (4) und des Kolbens (16) hervorzurufen.

6. Medizinische Vorrichtung (2) nach einem der Ansprüche 1 bis 5, wobei die vorgefüllte Spritze (3) eine Kolbenstange (18) umfasst, die in Translation fest mit dem Kolben (16) verbunden und beweglich mit Bezug auf den Spritzenkörper (4) gemäß der axialen Verschiebungsrichtung montiert ist, und der eine des ersten und zweiten Verpackungsteils (22, 23) konfiguriert ist, um die Kolbenstange (18) gemäß der axialen Verschiebungsrichtung bei der Verschiebung des ersten und zweiten Verpackungsteils (22, 23) aus der Lagerkonfiguration in die Öffnungskonfiguration zu verschieben.

7. Medizinische Vorrichtung (2) nach einem der Ansprüche 1 bis 6, wobei der erste Verpackungsteil (22) mindestens ein Rückhalteorgan (29) umfasst, das konfiguriert ist, um die vorgefüllte Spritze (3) auf dem ersten Verpackungsteil (22) zurückzuhalten.

8. Medizinische Vorrichtung (2) nach einem der Ansprüche 1 bis 7, wobei die vorgefüllte Spritze (3) einen Anschlussstutzen (5) umfasst, umfassend einen rohrförmigen Anschlussteil (6), der für den Durchgang des Fluids, das verabreicht werden soll, ausgelegt ist, und einen Verschluss (9), der konfiguriert ist, um ein freies Ende des rohrförmigen Anschlussteils (6) zu verschließen, wobei der Verschluss (9) an das freie Ende des rohrförmigen Anschlussteils (6) durch einen durchtrennbaren Bereich (11) verbunden ist.

9. Medizinische Vorrichtung (2) nach Anspruch 8, wobei die vorgefüllte Spritze (3) eine Schutzkappe (12) umfasst, die auf dem Anschlussstutzen (5) montiert ist.

10. Medizinische Vorrichtung (2) nach Anspruch 9, wobei die Schutzkappe (12) einen Kopplungsteil (15) umfasst, der an den Verschluss (9) derart gekoppelt ist, dass eine Drehung des Kopplungsteils (15) um eine Drehachse parallel zur Ausdehnungsrichtung des rohrförmigen Anschlussteils (6) einen Bruch des durchtrennbaren Bereichs (11) verursacht.

11. Medizinische Vorrichtung (2) nach Anspruch 10, wobei der zweite Verpackungsteil (23) mindestens ein Rückhalteelement (38) umfasst, das konfiguriert ist, um die Schutzkappe (12) zurückzuhalten, wobei die Verpackung (21) derart konfiguriert ist, dass die Verschiebung des ersten und zweiten Verpackungsteils (22, 23) aus der Lagerkonfiguration in die Öffnungskonfiguration einen Bruch des durchtrennbaren Bereichs (11), der den Verschluss (9) und den rohrförmigen Anschlussteil (6) verbindet, und ein Zurückhalten der Schutzkappe (12) auf dem zweiten Verpackungsteil (23) verursacht.

12. Medizinische Vorrichtung (2) nach Anspruch 11, wobei die Schutzkappe (12) mindestens ein Antriebselement (39) umfasst, das konfiguriert ist, um mit dem mindestens einen Rückhaltelement (38) zusammenzuarbeiten, wobei der zweite Verpackungsteil (23) und die Schutzkappe (12) beweglich in Rotation mit Bezug aufeinander derart montiert sind, dass das mindestens eine Rückhalteelement (38) geeignet ist, eine erste Winkelposition einzunehmen, in der das mindestens eine Rückhalteelement (38) winklig vom mindestens einen Antriebselement (39) versetzt ist, und eine zweite Winkelposition, in der das mindestens ein Rückhalteelement (38) mit dem mindestens einen Antriebselement (39) zusammenarbeitet, um den durchtrennbaren Bereich (11) zu brechen und die Schutzkappe (12) zurückzuhalten, wobei die Verpackung (21) derart konfiguriert ist, dass eine Verschiebung des ersten und zweiten Verpackungsteils (22, 23) aus der Lagerkonfiguration in die Öffnungskonfiguration eine Verschiebung des mindestens einen Rückhaltelements (38) aus der ersten Winkelposition in die zweite Winkelposition verursacht.

13. Medizinische Vorrichtung (2) nach Anspruch 3 oder einem der Ansprüche 4 bis 12, wenn er von Anspruch 3 abhängt, wobei die Verpackung (21) Führungsmittel umfasst, die konfiguriert sind, um eines des ersten und zweiten Verpackungsteils (22, 23) mit Bezug auf das andere des ersten und zweiten Verpackungsteils (22, 23) gemäß einer ersten schraubenförmigen Bewegung in einer ersten Aufrollrichtung bei einer Verschiebung des ersten und zweiten Verpackungsteils (22, 23) aus der Lagerkonfiguration in die Zwischen-Aktivierungskonfiguration und gemäß einer zweiten schraubenförmigen Bewegung in einer zweiten umgekehrten Aufrollrichtung mit Bezug auf die erste Aufrollrichtung bei einer Verschiebung des ersten und zweiten Verpackungsteils (22, 23) aus der Lagerkonfiguration in die Zwischen-Aktivierungskonfiguration zu führen.

14. Medizinische Vorrichtung (2) nach Anspruch 13, wobei die Führungsmittel einen Führungsdorn (37), der auf dem einen des ersten und zweiten Verpackungsteils (22, 23) vorgesehen ist, und eine Führungsnut (36) umfassen, die auf dem anderen des ersten und zweiten Verpackungsteils (22, 23) vorgesehen ist, wobei der Führungsdorn (37) gleitend in der Führungsnut (36) montiert ist.

15. Medizinische Vorrichtung (2) nach einem der Ansprüche 1 bis 10, wobei der erste und zweite Verpackungsteil (22, 23) schwenkend mit Bezug aufeinander um eine Schwenkachse zwischen der Lagerkonfiguration und der Öffnungskonfiguration montiert sind.

16. Medizinische Vorrichtung (2) nach Anspruch 6 und 15, wobei der zweite Verpackungsteil (23) ein Betätigungsorgan (42) umfasst, umfassend eine Nockenfläche (43), die konfiguriert ist, um die Kolbenstange (18) aus einer ersten Kolbenposition in eine zweite Kolbenposition beim Schwenken des ersten und zweiten Verpackungsteils (22, 23) aus der Lagerkonfiguration in die Öffnungskonfiguration zu verschieben.

17. Medizinische Vorrichtung (2) nach Anspruch 15 oder 16, wobei der erste Verpackungsteil (22) Blockierungsmittel umfasst, die konfiguriert sind, um axial den Spritzenkörper (4) auf dem ersten Verpackungsteil (22) zu blockieren.

18. Medizinische Vorrichtung (2) nach einem der Ansprüche 1 bis 17, wobei der zweite Verpackungsteil (23) auf abnehmbare Weise auf dem ersten Verpackungsteil (22) montiert ist.

19. Medizinische Vorrichtung (2) nach einem der Ansprüche 1 bis 18, die ein Trockenmittel und/oder einen Sauerstoffabsorber umfasst, der in der Verpackung (21) angeordnet ist.

20. Medizinische Vorrichtung (2) nach einem der Ansprüche 1 bis 19, wobei die vorgefüllte Spritze (3) auf sterile Weise in der Verpackung (21) angeordnet ist.

21. Medizinische Vorrichtung (2) nach einem der Ansprüche 1 bis 20, wobei die Verpackung (21) erste Montagemittel und zweite komplementäre Montagemittel der ersten Montagemittel umfasst, wobei die ersten Montagemittel der medizinischen Vorrichtung (2) konfiguriert sind, um mit den zweiten Montagemitteln einer benachbarten medizinischen Vorrichtung zusammenzuarbeiten, um die Montage der zwei medizinischen Vorrichtungen zu ermöglichen.

## Claims

1. A medical device (2), comprising:
- a pre-filled syringe (3) including a syringe body (4) and a piston (16) slidably mounted in the syringe body (4) in an axial direction of displacement, the syringe body (4) and the piston (16) delimiting an inner chamber (17) containing a fluid to be administered to a patient; and
- a package (21) in which the pre-filled syringe (3) is disposed, the package (21) including a first package portion (22) and a second package portion (23) movably mounted relative to each other between a storage configuration in which the first and second package portions (22, 23) prevent a removal of the pre-filled syringe (4) out from the package (21), and an opening configuration in which the first and second package portions (22, 23) enable a removal of the pre-filled syringe (3) out from the package (21),
**characterized in that** the medical device (2) is configured such that a displacement of the first and second package portions (22, 23) from the storage configuration to the opening configuration causes a displacement of the piston (16) of the pre-filled syringe (3) from a first piston position to a second piston position.

2. The medical device (2) according to claim 1, wherein the first package portion (22) includes a receiving housing (26) in which the pre-filled syringe (3) is at least partially disposed, and a passage opening (27) emerging into the receiving housing (26) and intended for the passage of the pre-filled syringe (3), the second package portion (23) being configured to at least partially close the passage opening (27) and to prevent a removal of the pre-filled syringe (3) out from the receiving housing (26) when the first and second package portions (22, 23) are in the storage configuration, and being configured to at least partially clear the passage opening (27) and to enable a removal of the pre-filled syringe (3) out from the receiving housing (26) when the first and second package portions (22, 23) are in the opening configuration.

3. The medical device (2) according to claim 1 or 2, wherein the first and second package portions (22, 23) are configured to occupy an intermediate activation configuration, the package (21) being configured such that a displacement of the first and second package portions (22, 23) from the storage configuration to the intermediate activation configuration causes a displacement of the piston (16) in the syringe body (4) in a first axial direction.

4. The medical device (2) according to claim 3, wherein the package (21) is configured such that a displacement of the first and second package portions (22, 23) from the intermediate activation configuration to the opening configuration causes a displacement of the piston (16) in the syringe body (4) in a second axial direction opposite to the first axial direction.

5. The medical device (2) according to claim 3 or 4, wherein one of the first and second package portions (22, 23) includes a first axial thrust surface (28) configured to transmit a thrust force to the piston (16), and the other of the first and second package portions (22, 23) includes a second axial thrust surface (35) configured to transmit a thrust force to the syringe body (4), the package (21) being configured such that a displacement of the first and second package portions (22, 23) from the storage configuration to the intermediate activation configuration causes bringing the first and second axial thrust surfaces (28, 35) together axially so as to result in a relative displacement of the syringe body (4) and the piston (16).

6. The medical device (2) according to any one of claims 1 to 5, wherein the pre-filled syringe (3) includes a piston rod (18) secured in translation with the piston (16) and movably mounted relative to the syringe body (4) in the axial direction of displacement, and one of the first and second package portions (22, 23) is configured to displace the piston rod (18) in the axial direction of displacement during the displacement of the first and second package portions (22, 23) from the storage configuration to the opening configuration.

7. The medical device (2) according to any one of claims 1 to 6, wherein the first package portion (22) includes at least one retaining member (29) configured to retain the pre-filled syringe (3) on the first package portion (22).

8. The medical device (2) according to any one of claims 1 to 7, wherein the pre-filled syringe (3) includes a connecting tip (5) including a tubular connecting portion (6) intended for the passage of the fluid to be administrated, and an obturator (9) configured to close a free end of the tubular connecting portion (6), the obturator (9) being connected to the free end of the tubular connecting portion (6) by a frangible area (11).

9. The medical device (2) according to claim 8, wherein the pre-filled syringe (3) includes a protective cap (12) mounted on the connecting tip (5).

10. The medical device (2) according to claim 9, wherein the protective cap (12) comprises a coupling portion (15) coupled to the obturator (9) such that a rotation of the coupling portion (15) about an axis of rotation parallel to the extension direction of the tubular connecting portion (6) causes a breakage of the frangible area (11).

11. The medical device (2) according to claim 10, wherein the second package portion (23) includes at least one retaining element (38) configured to retain the protective cap (12), the package (21) being configured such that a displacement of the first and second package portions (22, 23) from the storage configuration to the opening configuration causes a breakage of the frangible area (11) connecting the obturator (9) and the tubular connecting portion (6) and a retention of the protective cap (12) on the second package portion (23).

12. The medical device (2) according to claim 11, wherein the protective cap (12) includes at least one drive element (39) configured to cooperate with the at least one retaining element (38), the second package portion (23) and the protective cap (12) being mounted movable in rotation relative to each other such that the at least one retaining element (38) is adapted to occupy a first angular position in which the at least one retaining element (38) is angularly offset from the at least one drive element (39), and a second angular position in which the at least one retaining element (38) cooperates with the at least one drive element (39) so as to break the frangible area (11) and retain the protective cap (12), the package (21) being configured such that a displacement of the first and second package portions (22, 23) from the storage configuration to the opening configuration causes a displacement of the at least one retaining member (38) from the first angular position to the second angular position.

13. The medical device (2) according to claim 3 or any one of claims 4 to 12 when it depends on claim 3, wherein the package (21) includes guide means configured to guide one of the first and second package portions (22, 23) relative to the other of the first and second package portions (22, 23) according to a first helical movement in a first winding direction during a displacement of the first and second package portions (22, 23) from the storage configuration to the intermediate activation configuration, and according to a second helical movement in a second winding direction reversed with respect to the first winding direction during a displacement of the first and second package portions (22, 23) from the intermediate activation configuration to the opening configuration.

14. The medical device (2) according to claim 13, wherein the guide means include a guide lug (37) provided on one of the first and second package portions (22, 23), and a guide groove (36) provided on the other of the first and second package portions (22, 23), the guide lug (37) being slidably mounted in the guide groove (36).

15. The medical device (2) according to any one of claims 1 to 10, wherein the first and second package portions (22, 23) are pivotally mounted relative to each other about a pivot axis between the storage configuration and the opening configuration.

16. The medical device (2) according to claims 6 and 15, wherein the second package portion (23) includes an actuating member (42) including a cam surface (43) configured to displace the piston rod (18) from a first rod position to a second rod position during the pivoting of the first and second package portions (22, 23) from the storage configuration to the opening configuration.

17. The medical device (2) according to claim 15 or 16, wherein the first package portion (22) includes immobilizing means configured to axially immobilize the syringe body (4) on the first package portion (22).

18. The medical device (2) according to any one of claims 1 to 17, wherein the second package portion (23) is removably mounted on the first package portion (22).

19. The medical device (2) according to any one of claims 1 to 18, which includes a desiccant and/or an oxygen absorber disposed in the package (21).

20. The medical device (2) according to any one of claims 1 to 19, wherein the pre-filled syringe (3) is disposed in a sterile manner in the package (21).

21. The medical device (2) according to any one of claims 1 to 20, wherein the package (21) includes first assembly means and second assembly means complementary to the first assembly means, the first assembly means of the medical device (2) being configured to cooperate with the second assembly means of an adjacent medical device so as to allow the assembly of the two medical devices.
